# EUROPEAN PATENT APPLICATION

(11) **EP 2 955 522 A1**
(43) Date of publication of application: **16.12.2015**
(21) Application number: 14382224.5
(22) Date of filing: 13.06.2014
(51) Int. Cl.: G01N 33/574, C12Q 1/68

(54) **A method for monitoring the treatment of patients with tumors expressing EGFR**

(71) Applicant: Fundació Privada Institut d'Investigació Oncològica de Vall Hebron, 08035 Barcelona (ES)
(72) Inventor: Villanueva Cardus, Josep, 08035 Barcelona (ES); Katsila, Theodora, 08035 Barcelona (ES); Tabernero Caturla, Josep Maria, 08035 Barcelona (ES)
(74) Representative: Cueto, Sénida

(57) **Abstract**

The invention refers to an *in vitro* method to monitor the response and resistance to a treatment of a patient suffering from an EGFR expressing tumor, with an anti-cancer agent against an anti-EGFR expressing tumor, the method comprising:
- determining the level of pEGFR in a body fluid sample taken from a patient suffering from an EGFR expressing tumor, and
- comparing said level with a reference value, the reference value being the level of pEGFR in a sample taken from the same patient before starting said treatment, and wherein the increase in said level is indicative of response to the treatment, and wherein the decrease in said level is indicative of the appearance of resistance to the treatment,

and a kit.

## Description

### Background of the invention

A major obstacle in the treatment of cancer patients is the lack of secreted biomarkers of response and primary/secondary resistance to therapy that could NOT be monitored by non-invasive tests. The identification of specific and easily assayed secreted biomarkers for anti-cancer agents for the prediction and monitoring of response and resistance to therapy is of paramount importance.

The search for secreted biomarkers that can guide the therapeutic treatment of choice is typically performed using *in vitro* experimental model systems. Blood-based tumor biomarker detection has proven to be difficult especially due to the relatively low abundance of tumor-specific biomarkers.

The cell cancer secretome has emerged as a new approach for the proteomics-based investigation of tumorigenesis and responses to novel targeted therapies that avoid the limitations of blood-based profiling, such as the difficulties of large-scale characterization of the human plasma proteome, that shows that <10% of the 'core' plasma proteome (estimated to contain at least 10,000 proteins) is being effectively sampled with current approaches, a small fraction and one that principally includes proteins at higher abundance (>1 µg/ml); some proteins that vary in abundance with the presence or absence of disease are not likely be detected, or will have difference signals too subtle for the semiquantitative methods used for initial analysis. Also,unless specially assayed, proteins that do not vary in abundance but vary as cleavage products or according to post-translational modifications are also likely to be missed (14).

It has been shown that cancer cell line secretomes contain proteins that might help in monitoring critical aspects of cancer progression and therapeutics, and they can particularly help in tumor biomarker discovery.

Tumors expressing the Epidermal Growth Factor Receptor (EGFR) are numerous and of great relevance. EGFR is a receptor belonging to the family of the proteins with tyrosine kinase activity, and is expressed in different types of cancer, for example, in head and neck, ovarian, cervical, bladder, oesophageal, gastric, breast, endometrial, non-small cell lung cancer (NSCLC), and colorectal cancer (CRC), wherein disease initiation and progression is fundamental. EGFR-targeted therapies based on inhibiting the receptor tyrosine kinase activity of the EGFR and blocking the EGFR ligand binding with different agents, such as monoclonal antibodies are being used in the clinic.

It is known that patients who initially benefit from EGFR-targeted therapies eventually develop resistance (3-11). Besides this fact, intensive clinical data established that KRAS mutations confer resistance to certain anti-cancer agents, for example certain monoclonal antibodies, such as Cetuximab (a monoclonal antibody anti-EGFR which binds to EGFR with a high specificity and blocks ligand-induced phosphorylation of the receptor) used in patients with EGFR expressing tumors, such as CRC patients. KRAS mutations are also among the preferred mechanisms of acquired resistance to Cetuximab treatment (1, 2), though an interesting twist came recently when it was shown that not all the tumors carrying KRAS mutations were equally resistant to Cetuximab action (12, 13).

The active form of EGFR is pEGFR, wherein the EGFR molecule is phosphorylated in the tyrosine residues of its C-terminal end. A treatment with an anti-EGFR agent is expected to low the pEGFR level as the activated form of the receptor, with respect to the level at the beginning of the treatment. pEGFR has been found in tissue samples and cells.

The amount of pEGFR in a cell does not serve as a biomarker. Therefore, it was not known until now that pEGFR could serve as a proof of the response or resistance of a patient suffering from cancer, more specifically from an EGFR expressing tumor, to a treatment with an anti-cancer drug, more specifically an anti-EGFR drug, as a monoclonal antibody, and more specifically, Cetuximab.

A problem of the state of the art is that these receptor proteins as EGFR are not only rarely found in fluid samples as plasma, but if they are present at all, they are present not as the whole protein, but only a fragment, or fragments thereof. The reason for that is that they have frequently been cut through, for example, the mechanism called *shedding,* whereby a protease cuts the extracellular domain of a receptor and said domain can then freely circulate. Also, a complete receptor is very rarely secreted for another reason, which is the lack of stability, and if it secreted, this occurs under a vesicle form. On the basis of these facts, the possibility of determining amounts of said proteins in fluids, such as plasma, is even lower.

There is an example of a mutant form of EGFR secreted in glioblastoma, which is form EGFRvIII, but it is secreted in a constitutive form and what can be seen with chemotherapy is that the EGFRvIII secreted in vesicles decreases. There is not an active secretion mechanism is the case of glioblastoma. The situation is completely different from the findings that led to the present invention. The activated pEGFR (phosphorylated in a Tyr residue) able to induce cellular proliferation, has never been found to be secreted as the complete molecule.

A second problem of the state of the art is that the resistance to certain drugs as Cetuximab is currently measured through imaging tests, and in order to know whether a patient suffering from cancer, more specifically an EGFR expressing tumor, is developing, or has developed, a resistance against an anti-cancer drug, one has to wait sufficient time for the tumor to grow again. Also an additional drawback of the imaging methods is that they frequently involve the administration of radioactive isotopes to the patient previous to the medical test.

A third problem is that in some cases a drug, as Cetuximab is not efficient in a patient suffering an EGFR tumor, particularly CRC, against said tumor, from the very beginning of the treatment, or after a time period of treatment, ceasing to be effective at a specific moment. There is not a solution to this situation whereby the moment at which the treatment looses its efficiency can easily be determined

### Problems solved by the present invention

All three problems have been solved by the present invention, and specifically through the facts that:
- pEGFR has surprisingly been found in a fluid sample, more specifically in a plasma sample, moreover,
- it has been found to be present in a plasma sample as a complete molecule,
- the secreted biomarker, pEGFR is the target molecule of the anti-cancer drug, and
- differences in the pEGFR amounts at different times for a patient can be measured.

The determination of the pEGFR level in a rapid and non-invasive way allows the detection of these patients with a lack of response, or resistance development, to an anti-cancer agent, more specifically, an EGFR expressing tumor. The mentioned determination is based on the findings that: as long as the drug, as Cetuximab, is being effective, the level of pEGFR in plasma would be higher, with respect to the level for said patient before starting the treatment, and as soon as the level of pEGFR in plasma decreases with respect to the level for said patient before starting the treatment, it means that the drug has ceased being effective. The possibility offered by the present invention of measuring a difference in pEGFR level from a plasma sample solves the problem of how to determine the progression an EGFR expressing tumor in an easy, non-invasive way.

One of the most striking features found by the inventors is that the secreted biomarker is the target of the anti-cancer drug, and that the secretion correlates with the response to the anti-cancer drug. The p(Y1068)EGFR increases its level in a plasma sample when the patient responds to a treatment with an anti-cancer drug, as Cetuximab, and the secretion decreases when they cease to respond to an anti-cancer drug, as Cetuximab. The limited response or lack of response to Cetuximab is correlated according to present invention with the presence of mutations in KRAS gene, and partiularly the KRAS-G12V and KRAS-G13D mutational statuses are correlated to no-response and limited response to Cetuximab, respectively (12).

It has been proved that 3D-spheroids from CRC cells generate secretomes with a drug sensitivity profile that correlates well with the response of CRC patients to the drug, illustrating molecular connections between intracellular and extracellular signalling in tumor cells

pEGFR can be used as a secreted biomarker of response to Cetuximab, that can be measured from a fluid sample, such as urine, cerebrospinal fluid, saliva, tumor interstitial fluid, blood, serum or plasma sample, and more preferably from a blood-derived sample like a plasma sample of a patient, or derivatives of any of them, and compared with a reference value of pEGFR being the "reference value " the level measured before starting the treatment, for said patient.

The method provided by the present invention comprising determination of pEGFR in a sample like a plasma sample solves the mentioned problems and has additional advantages as hereinbelow:
- it is more favourable in economic terms since it involves lower costs than the currently used imaging tests,
- it can be more frequently done, resulting in an earlier detection of resistance against a treatment with this drug, or the appearance of recurrences.
- the test can be done before a particular tumor increases its size again upon appearance of a resistance to a treatment with a drug, avoiding long waiting times, that would result in a problematic progression of the disease,
- and especially it is much better from the patient's view point due to its non-invasive character.

It is therefore the first time that secreted pEGFR has been detected and it is the first time that it has been found that the secretion correlates with the response to a drug as Cetuximab.

### Description of the invention

The following terms are defined herein below for a better understanding of the present invention:
"Response": A response to a treatment means that the tumor decreases its size,
"resistance": A resistance to a treatment means that the tumor continues growing, or the tumor does not decrease its size,
"anti-cancer drug", "drug" and "anti-cancer agent" are terms used indistinctly in the present application,
"biofluid sample" or "body fluid sample" means a sample from liquids originating from inside the bodies of living patients. They include fluids that are excreted or secreted from the body such as a sample from: saliva, urine, interstitial tumor fluid, cerebrospinal fluid, blood, blood derived sample, for example whole blood, serum, plasma, or derivatives thereof, wherein "derivatives thereof" means samples from any of the mentioned fluids that could have been previously manipulated or treated in a manner appropriate for the use in the method of the invention,
"ligand": in protein-ligand binding, a ligand usually is a signal-triggering molecule, binding to a site on a target protein,
EGFR: it is a transmembrane receptor composed of an intracellular tyrosine kinase domain with an autophosphorylation site; a transmembrane domain; and an extracellular EGF ligand-binding domain. It is activated by binding of its specific ligands, including epidermal growth factor and transforming growth factor α. Following ligand binding, a conformational change occurs in the EGFR monomers. This leads to EGFR dimerization, that brings the two monomers into close proximity, stimulating the kinase activity of EGFR, provoking trans-autophosphorylation on multiple tyrosine residues in C-terminal end of the EGFR molecule.

pEGFR or phosphorylated-EGFR: pEGFR stands for phosphorylated epidermal growth factor receptor and is the activated form of EGFR. In the context of the present invention pEGFR is p(Y1068)EGFR, this means, EGFR phosphorylated in, at least, position Tyr 1068. p(Y1068)EGFR can also be phosphorylated in additional Tyr positions. Therefore what is measured according to the present invention by means of ELISA and WB in conditioned media in cells and plasma is p(Y1068)EGFR.

"reference value" means the level of pEGFR in a body fluid sample taken from a patient before starting an treatment against an EGFR expressing tumor, and wherein the later decrease in said level is indicative of the lack of response or appearance of resistance to the treatment.

"level" and "amount" are used indistinctly in the present application, unless otherwise indicated.

KRAS, or K-RAS: is a protein also known as *GTPase KRas* or *V-Ki-ras*2 *Kirsten rat sarcoma viral oncogene homolog,* that in humans is encoded by the *KRAS* gene The KRAS gene belongs to the oncogenes that when mutated, have the potential to cause normal cells to become cancerous. The protein is involved primarily in regulating cell division. As part of a signaling pathway known as the RAS/MAPK pathway, the protein relays signals from outside the cell to the cell's nucleus.

KRAS-G12V: is the mutated KRAS gene wherein glycine has been replaced by valine at codon 12 (G12V),

KRAS-G13D is the mutated KRAS gene wherein glycine has been replaced by aspartic acid at codon 13 (G13D).

In a first aspect, the present invention relates to an *in vitro* method for monitoring the response or resistance to a treatment of a patient suffering from an EGFR expressing tumor, the method comprising:
- determining the level of pEGFR in a body fluid sample taken from a patient suffering from an EGFR expressing tumor, and
- comparing said level with a reference value, the reference value being the level of pEGFR in a sample taken from the same patient before starting said treatment, and wherein the increase in said level is indicative of response to the treatment, and wherein the decrease in said level is indicative of the appearance of resistance to the treatment.

The level of pEGFR in the method of the present invention can be measured by any method known in the art, without limitation, such as western blot, protein immunostaining, protein immunoprecipitation, immunoelectrophoresis, immunoblotting, BCA (Bicinchoninic Acid) assay, spectrophotometry, mass spectrometry, or immunochemistry methods suchg as enzyme assay (ELISA).

The anti-cancer agent can be an agent with the capacity of inhibiting the tyrosine kinase activity of a protein receptor, such as an agent against EGFR, for example an inhibitor of the Tyr kinase activity of EGFR, and more specifically a monoclonal antibody or a fragment of a monoclonal antibody. According to a preferred embodiment of the invention the monoclonal antibody is Cetuximab.

According to particular embodiments of the *in vitro* method of the invention, the cancer is an EFGR expressing tumor, preferably selected from the group comprising head and neck, ovarian, cervical, bladder, oesophageal, gastric, breast, endometrial, non-small cell lung cancer (NSCLC), and colorectal cancer. In a particularly preferred embodiment the EGFR expressing tumor is colorectal cancer, CRC, preferably, metastatic colorectal cancer.

CRC refers to any type of colorectal cancer. The most common type of colorectal cancer is adenocarcinoma. Other colorectal cancers include gastrointestinal carcinoid tumors, gastrointestinal stromal tumors, primary colorectal lymphoma, leiomyosarcoma, melanoma and squamous cell carcinoma.

The body fluid sample taken from the patient according to the invention can be selected from the group comprising saliva, urine, interstitial tumor fluid, cerebrospinal fluid, blood, blood derived sample, for example whole blood, serum, plasma, and a sample from a derivative of any of them. The sample is preferably a plasma sample.

According to the method of the invention, the sample can be taken from a patient suffering from an EGFR expressing tumor, more preferably CRC:
- before and during the administration of said anti-cancer agent, preferably an anti-EGFR agent, more preferably, an anti-EGFR monoclonal antibody, even more preferably Cetuximab.

The *in vitro* method of the invention can further comprising taking a decision on the treatment with the anti-cancer agent, wherein the treatment is continued if the level of pEGFR is at least as high as the reference value, or the treatment is discontinued if the level of pEGFR is lower than the reference value.

In a second aspect, the present invention relates to an *in vitro* method for selecting a patient suffering from an EGFR expressing tumor, for an anti-cancer treatment, preferably EGFR expressing tumors, more preferably cancer selected from the group comprising head and neck, ovarian, cervical, bladder, oesophageal, gastric, breast, endometrial, non-small cell lung cancer (NSCLC), and colorectal cancer, comprising determining in a body fluid sample taken from said patient the level of pEGFR, and wherein the decrease in said level means selecting the patient for the cancer treatment.

In a third aspect, the present invention relates to an anti-cancer agent for the treatment of a patient suffering from cancer, wherein the patient is selected using the method of the second aspect.

In a particular preferred embodiment of the method, the EGFR expressing tumor is colorectal cancer, the anti-EGFR monoclonal antibody is Cetuximab, and the sample is a plasma sample. In a fourth aspect, the present invention relates to a kit for *in vitro* monitoring the response or resistance to a treatment of a patient suffering from an EGFR expressing tumor, with an anti-cancer agent, comprising reagents suitable for determining the level of pEGFR in a body fluid sample taken from a patient, preferably a sample selected from the group comprising saliva, urine, interstitial tumor fluid, cerebrospinal fluid, blood, blood derived sample, for example whole blood, serum, plasma, or a sample from derivatives of any of them, said patient suffering from an EGFR expressing tumor, for example, head and neck, ovarian, cervical, bladder, oesophageal, gastric, breast, endometrial, non-small cell lung cancer (NSCLC), and colorectal cancer, and still more preferably, colorectal cancer. A particular preferred embodiment of the kit is a kit wherein the sample is a plasma sample, the anti-cancer agent is Cetuximab and the cancer is CRC.

According to the invention pEGFR is p(Y1068)EGFR, this means, EGFR phosphorylated in, at least, position Tyr 1068.

The invention also refers to the use of a kit as defined in the preceding paragraph for *in vitro* monitoring the response or resistance to a treatment of a patient suffering from an EGFR expressing tumor, with an anti-cancer agent according to the *in vitro* method of the invention.

In a fifth aspect, the present invention relates to the use of said kit for the diagnosis or prognosis of the appearance of therapy resistance in a patient suffering from an EGFR expressing tumor, or for selecting a patient suffering from an EGFR expressing tumor, for an anti-cancer treatment. The therapy resistance is a resistance against an anti-cancer agent, preferably an anti-EGFR agent, more preferably against a monoclonal antibody against EGFR and still more preferably against Cetuximab. According to a particularly preferred embodiment of the kit, it is used for the diagnosis or prognosis of the appearance of therapy resistance against Cetuximab of a patient suffering from CRC and the appearance of therapy resistance is measured in a plasma sample of said patient.

In a sixth aspect, the present invention relates to the use of a reagent for detecting pEGFR in a plasma sample through the *in vitro* method of the invention, as defined above. Said reagent can be for example an antibody, more preferably a monoclonal antibody.

Based on the analysis of plasma of patients undergoing Cetuximab treatment it has been found that the secretion of pEGFR correlates with the sensitivity of CRC cells to Cetuximab and the response of CRC patients to the drug. Therefore pEGFR can be used as a biomarker of response to Cetuximab treatment.

From the point of view of the scientific development underlying the invention, a methodology for 3D-spheroids, and also for 2D-culture cell-based assays and proteomics is developed, showing that protein secretion in 3D differs to that in 2D. 2D-cell culture conditions proved not to be as robust as the 3D-model of the present invention in recapitulating the clinically observed sensitivity/ resistance to Cetuximab in CRC patients. Using 3D-cell culture, the present invention demonstrates how the stimulation and blockage of the EGFR pathway in CRC cells is translated into an EGFR-centric protein secretion. An EGFR pathway-centric secretome induced by Cetuximab on 3D-spheroids of CRC cells is developed according to the present invention.

Isogenic cell lines harboring key mutations related to drug response/ resistance represent the ideal model to study the effect of molecular alterations since the mutation is isolated from the genetic background.

Parental (KRAS wild-type) SW48 cells are engineered using rAAV-mediated homologous recombination to create a panel of clones harbouring different mutant KRAS variants at the endogenous locus, but otherwise share the same genetic background. These endogenously engineered isogenic systems allow for unambiguous triangulation of specific genotype-phenotype interactions, while retaining the specific locus normal versus mutant context of target patients (20). An added value of the isogenic cell lines chosen herein is that they not only represent a real molecular scenario in the treatment of naive patients to Cetuximab, but they similarly provide a good model to study acquired resistance. It has been found that performing secretome-based proteomics on isogenic CRC cells sharing the KRAS-mutations found on patients yield secreted biomarkers in the clinical setting.

Considering the outcome of clinical data that show the strong correlation between mutated-KRAS and a lack of response to Cetuximab therapy, the inventors focused on the KRAS-G12V and KRAS-G13D mutational statuses, since they are correlated to no-response and limited response to Cetuximab, respectively (12).

It has been proved that 3D-spheroids from CRC cells generate secretomes with a drug sensitivity profile that correlates well with the response of CRC patients to the drug, illustrating molecular connections between intracellular and extracellular signalling in tumor cells.

The present invention also refers to the characterization of the protein secretion involved in the response and resistance to Cetuximab therapy, and its exploitation for biomarker discovery. The present invention proves that 3D-spheroids generating the 3D-secretomes have a drug sensitivity profile that correlates well with xenografts and CRC patients, as previously observed (12,22).

The unexpected finding of the partial sensitivity of the KRASG13D mutation found in 3D opened up a new therapeutic avenue previously closed for these CRC patients. The 3D-spheroids, but not the 2D-cell culture format, set apart the two KRAS mutations studied herein under the drug exposure.

Herein, the SW48 isogenic cell line is overall less sensitive to Cetuximab in 3D than in 2D, and clinically important differences based on the KRAS mutational variant status were now revealed. This is even reflected at the secretome level, where the number of the significant differences arising from the secretome comparison upon treatment with Cetuximab in the three cell lines is much smaller in spheroids than in 2D. All together, the dependence of cancer drug action on the cell culture format used is underlined, possibly explaining the discrepancies observed between the discovery studies *in vitro* and the lower efficacy of cancer drugs when tested in more physiological scenarios.

According to the invention, the treatment of CRC cells with Cetuximab in 2D showed a broad effect on different signaling pathways, most of them not directly related to the EGFR pathway. On the contrary, the Cetuximab-treated KRAS-wild type cells growing as 3D-spheroids showed a clear regulation in protein secretion related to the EGFR pathway. Several of the molecules regulated in the 3D-spheroids, in the presence of Cetuximab, are regulated in patients treated with anti-EGFR targeted therapy either in CRC or lung cancer (27-28).

It has also been found that the fact of pEGFR being secreted from CRC cells treated with Cetuximab, particularly in the 3D-spheroid setting, coincides with an increased amount of microvesicles in the CRC secretome. The pEGFR shedding observed herein does play a role as a modulator of the sensitivity to the drug. The fact that pEGFR is not increased upon drug treatment in KRAS-mutant cells could be related to the diminished importance of EGFR once the pathway is constitutively activated via KRAS mutations.

The present invention has the following advantages over the state of the art:
- it allows the monitoring of the anti-EGFR treatment against an EGFR expressing tumor, more specifically EGFR expressing tumours as CRC, through a non-invasive test
- it allows to guide the therapeutic treatment of choice and making amendments if appropriate
- it demonstrates the relevance of the 3D cultures for cancer research that has been previously underscored, as it represents a more physiologic approach allowing a better modelling of the therapeutic treatment investigated than in 2D (23-25).

### Brief description of the figures

Figure 1 shows the Secretome proteomics of SW48 KRAS-wild type cells in 2D and 3D cell culture. Figure 1A shows clear-cut differences between the secretomes from the two cell culture formats. Figure 1B shows a large proportion of proteins oversecreted in 3D-spheroids as compared to the 2D monolayer.
Figure 2 shows the Cetuximab effect on cellular proliferation of SW48 KRAS-wild type and KRAS-mutant isogenic cell lines in 2D and 3D cell culture. Figure 2A shows the results in 2D and Figure 2B shows the results in 3D format.
Figure 3 shows the Secretome Proteomics of Sensitivity to Cetuximab in 2D and 3D.
Figure 3A. Heat maps representing the proteins that were significantly over- and/or down secreted, when SW48 parental, KRAS-G13D and KRAS-G12V cells were grown in the 2D or 3D format and treated with EGF or EGF/Cetuximab (first biological replicate).
Figure 3B illustrates equal concentrations of secretomes (15 µg) resolved by SDS-PAGE and western blotted against TSG101, an stablished exosome marker.
Figure 3C represents Venn diagrams showing the significant proteins that differ between the two cell culture formats (2D and 3D) upon Cetuximab treatment, when comparing the dataset of KRAS-wild type status to the dataset of KRASG13D and that of KRAS-G12V status.
Figure 4 shows how the Cetuximab action in SW48 cells differentially regulates the EGFR pathway-linked secreted response in 3D-spheroids as compared to the 2D-cell culture.
Figure 4A shows an Ingenuity Pathway Analysis of the proteins that are differentially secreted upon EGF and EGF/ Cetuximab treatment of SW48 parental cells in 3D (two biological replicates, three technical replicates).
Figure 4B shows a Western-blot analysis of secretome proteins such as pEGFR, c-Met, CTSL2, KLK5 and SOD2.
Figure 4C shows a Western-blot analysis of cell lysates showing the activation of the EGFR pathway.
Figure 5 shows that pEGFR secretion is validated in secretomes and in the plasma of patients undergoing Cetuximab treatment.
Figure 5A shows the pEGFR levels detected by ELISA in the secretomes of SW48 parental and KRAS-G12V cells grown in 3D and compared to their counterparts in cell lysates.
Figure 5B shows the pEGFR levels detected by ELISA in the plasma of patients undergoing Cetuximab treatment.
Figure 5C shows CT scans (CT = computer tomography) of patients COLT014 and COLT019.

### Examples

All chemicals were purchased from Sigma-Aldrich (Madrid, Spain) unless stated otherwise.

### Patient eligibility and study design

All patients (see characteristics of patients on Table S1) were diagnosed of metastatic colorectal cancer with KRAS (exon 2) wild type status and treated with the standard weekly schedule of Cetuximab (400 mg/m² loading dose followed by 250 mg/m² weekly) in combination with the standard irinotecan-based cytotoxic regimen FOLFIRI (irinotecan 180 mg/m² day 1, leucovorin 400 mg/m² day 1, bolus 5-fluoruracil 400 mg/m² day 1 and infusional 5-fluorouracil 2400 mg/m² in 48 hours starting day 1, repeated every 2 weeks), until progression of the disease or unacceptable toxicity. Response evaluation was planned every eight weeks. All patients were treated in the first-line setting except for patient COLT016 that was treated in the second-line after failing oxaliplatin-based chemotherapy (mFOLFOX6). Blood samples for the serum proteomic analysis were obtained at baseline (before the treatment was initiated), and thereafter every eight weeks at the same time the response was evaluated.

**Table S1**

| **Characteristic** | **All patients (n=6)** |
|---|---|
| **Age** | |
| median (yr) | 62 |
| range (yr) | 47-74 |

| **Sex** | |
|---|---|
| female | 3 |
| male | 3 |

| **ECOG** | |
|---|---|
| 0 | 0 |
| 1 | 6 |
| 2 | 0 |

| **Site of primary cancer** | |
|---|---|
| Colon only | 6 |
| Rectum only | 0 |
| Colon and rectum | 0 |

| **Metastases** | |
|---|---|
| hepatic | 3 |
| hepatic and lung | 2 |
| ovary | 1 |

### Cell culture of differentiated cells and spheroids

The human colon adenocarcinoma cell line SW48 (*KRAS*-wild type) and the isogenic SW48 KI G12V and SW48 KI G13D (*KRAS*-mutants) cells were obtained from Horizon Discovery Ltd, and cultured in 5% CO₂ and 95% humidified atmosphere air at 37°C in Dulbecco's modified Eagle's medium: Nutrient Mixture F-12 (DMEM/F12; Invitrogen, Carlsbad, CA), supplemented with 10% fetal bovine serum (FBS; Invitrogen, Carlsbad, CA), and 2 mM L-Glutamine (Invitrogen, Carlsbad, CA). For spheroids, SW48 cells meeting the viability limit of 90% were inoculated in T25 ultra-low attachment cell culture flasks (Corning Life Sciences, Aston, MA) in a growth medium - colonosphere medium - consisting of DMEM/F12, penicillin/streptomycin (100U/100 g/mL; Life Technologies, Carlsbad, CA), B27 (1:50; Gibco), heparin sodium salt (4 µg/mL), non-essential amino acids (1:100; Gibco), sodium pyruvate (1:100; Gibco), L-Glutamine (2mM; Invitrogen, Carlsbad, CA), human recombinant FGF-2 and EGF (at 10 and 20 ng/mL, respectively; Peprotech, Rocky Hill, NJ), D-Glucose (60 mg/mL), apotransferrin (1 mg/mL), insulin (0.25 mg/mL), putrescin (96 g/mL), sodium selenite (52 ng/mL) and progesterone (63 ng/mL). On day 3, spheroids bloomed from clusters of cells with strong evidence of cell metabolism and two-thirds of their culture medium was renewed, following low-speed centrifugation.

### Secretome Sample Preparation in 2D and 3D.

On day 1, SW48 cells were plated in 2D, 15 cm dishes (4x10⁶ cells/dish) and grown for 48h in DMEM/F12 supplemented with 10% FBS and 2 mM L-Glutamine (day 1). On day 3, cells were washed fice times and conditioned media were collected after 24h (day 4), followed by cell number and viability measurements (Countess Invitrogen, Carlsbad, CA). To form spheroids, 4x106 SW48 cells previously grown in 2D were inoculated in T25 ultra low attachment cell culture flasks (Corning Life Sciences, Aston, MA) in colonosphere medium, after meeting the viability limit of 90% (day 1). On day 3, spheroids bloomed from clusters of cells with strong evidence of cell metabolism and hence, two-thirds of their culture medium were renewed. On day 6, cells were washed five times, twice with 5 mL of PBS and three times with 5 mL of serum---free DMEM/F12 medium (pelleted by low-speed centrifugation). To estimate the total cell number per flask, a 1 mL aliquot of spheroids (taken from a T25/T75 flask of cells which was shaken to randomly distribute the spheroids was removed prior to washing and a single cell suspension was achieved using trypsin digestion followed by mechanical dissociation. Following neutralization of trypsin, cells were pelleted by low-speed centrifugation adn then the cell number and viability were determined using Countess (Invitrogen Carlsbad, CA). Conditioned media (30 ml) were collected at 24h (day 7), after spinning down the cells (200 g, 1600 rpm, 5 min). The supernatants were collected, filtered through a Millex.GP 0.22 pore syringe driven filters, (Millipore, Ireland) to remove cell debris and concentrated using centrifugal filters, with a 10-kd cutoff (Millipore Amicon Ultra; Millipore) first at 4,000 g at room temperature, until a final volume of 250 µK and then at 14,000g at room temperature until a final volume of 50 µK (10-Kd cutoff, Millipore Microcon; Millipore). Protein amounts were determined using a Pierce BCA protein assay kit (Thermo Scientific). Cell number and viability measurements were performed using Countess (Invitrogen, Carlsbad, CA). The downstream processing of the secretome samples has been also described in recent reports by the inventors (16, 21).

### LC-MS/MS analysis

Samples were in-solution digested previous to analysis by liquid chromatography-mass spectrometry (LC-MS) as described in (3). Following the completion of digestion, samples were zip-tipped, dried and re-dissolved in 30 % CAN (acetonitrile), 0.1 & FA (formai acid) to a final concentration of 1 µg/µL prior to LC-MS analysis. Samples were analysed using a LTQ Velos-Orbitrap mass spectrometer (Thermo Fisher Scientific, Bremen, Germany). The instrument control was perfomred using an EASY-nLC system (Proxeon Biosystems, Thermo Fisher Scientific) with a two-linear-column system. Digests were loaded onto a trapping guard column (EASY-column, 2 cm long, ID 100 µm and packed with Reprosil C18, 5 µm particle sized from Proxeon Biosystems, (Thermo Fisher Scientific). Separation was achieved by using a mobile phase from 0.1 % FA (Buffer A) and 100 % ACN with 0.1 % FA (Buffer B) and applying a linerar gradient from 5 % to 35 % buffer B for 120 min or 240 m in (depending on the experimental setup) at a flow rate of 300 nL/min, Ions were generated applying a voltage of 1.9 kV ta a stainless steel nano-bore emitter (Proxeon Biosystems, Thermo Fisher Scientific), connected to the end of the analytical column.

The LTQ Velos-Orbitrap mass spectrometer was operated in data-dependent mode. A scan cycle was initiated with a full-scan MS spectrum (from m/z of 300 to m/z of 1600) acquired in the Orbitrap with a resolution of 30,000. The twenty most abundant ions were selected for CID fragmentation in the linear ion trap (LTQ), when their intensity exceeded a minimum threshold of 1,000 counts, excluding single charged ions. Accumulation of ions for both MS and MS/MS scans was perfomred in the linear ion trap, and the AGC target values were set to 1x10⁶ ions for survey MS and 5,000 ions forMS/MS experiments. The maximum ion accumulation time was 500 and 200 msec in the MS and MS/MS modes respectively. The normalize dcollision energy was set to 35 %, and one microscan was acquired per spectrum. Ions subjected to MS/MS with a relative mass windows of 10 ppm were excluded from further sequencing for 20 sec. For all precursor masses, a window of 20 ppm and isolation width of 2 Da were defined. Orbitrap measurements were performed enabling the lock mass option (m/z 445,120024(for survey scabs to improve mass accuracy.

### Protein identification

The Xcalibur 2.1.0 software (Thermo Fisher Scientific) was used to generate RAW files of each MS run. The RAW files were processed using Proteome Discoverer 1.4 (Thermo Fisher ScienQfic; version 1.4.0.288). All secretome MS/MS were searched against the human database Swiss-Prot_2012.06 (20312 entries). In all cases, the search engine used was MASCOT (Matrix Science, London, U.K.; version 2.3.02). The data was searched with a fragment ion mass tolerance of 0.8 Da and a parent ion tolerance of 10 ppm. Oxidation of methionine and carbamidomethylation of cysteines were specified in MASCOT as dynamic and static modification respectively, and one missed cleavage was allowed for tryptic cleavage. The files generated from MASCOT (.DAT files) were then uploaded into Scaffold (Proteome software, Inc., Portland, OR; version 4.0.5) resulting in a non-redundant list of identified proteins per sample. Peptide identifications were accepted if they could be established at greater than 90.0% probability by the Peptide algorithm according to (8). Further details on the protein identification and statistics have been published in (10, 15, 19, and 29)

### Treatment regime.

Herein, both differentiated SW48 cells and spheroids were treated with EGF (2 ng/mL), following an 18h serum starvation (day 4/ day 7). On day 5/ day 8, their conditioned media were collected (t = 24h). Upon Cetuximab treatment, cells were treated with EGF (2 ng/mL, 10 min), followed by 0.5 mg/mL of Cetuximab (day 4/ day 7). After 24h, the Cetuximab-treated cells were washed and their conditioned media were collected after 24h (day 6/ day 9). Cell number and viability measurements were performed at days 5 (EGF-treated cells) and 6 (Cetuximab-treated cells) for the differentiated cells and days 8 (EGF-treated cells) and 9 (Cetuximab-treated cells) for the spheroids.

### Western Blotting.

Differentiated SW48 cells and spheroids were seeded/inoculated as described, and allowed to grow at the specified times and test conditions. Western blot analysis was performed using the rabbit polyclonal antibodies to pEGFR and phosphorylated Erk (pErk) (1/2) and the rabbit monoclonal antibody to phosphorylated Akt (pAkt) - all obtained from Cell Signaling Technology, Inc. (Danvers, MA). The mouse monoclonal antibodies to c-Met, KLK5, CTSL2 and TSG101 were purchased from Abcam (Cambridge, UK). The rabbit polyclonal antibody to SOD-2 was obtained from Santa Cruz Biotechnology, Inc. (Santa Cruz, CA). Sheep anti-mouse and donkey anti-rabbit IgG horseradish peroxidase-conjugated secondary antibodies were purchased from GE Healthcare (Amersham, UK). Densitometry was carried out using the Image J software (National Institutes of Health, Bethesda, MD).

### Phosphorylated EGFR ELISA.

The 3D-secretomes and -cell lysates of SW48 KRAS-wild type and KRAS-G12V cells (upon treatment of EGF and EGF/Cetuximab) and plasma samples from patients were used for pEGFR detection using the Enzyme Linked Immunosorbent Assay (ELISA) system (PathScan® Phospho-EGF Receptor (Tyr1068) Sandwich ELISA Kit, Cell Signaling Technology) according to the manufacturer's specifications. Relative quantification occurred as advised by the manufacturer, following sample normalization by protein amount and then, comparing the relative target abundance by comparing signal intensities.

### Experiments to find the sensitivity or resistance of KRAS-wild type versus KRAS mutated isogenic CRC cell lines to Cetuximab

To establish a framework to characterize the Cetuximab-induced protein secretion in CRC cells, the sensitivity or resistance of KRAS-wild type versus KRAS mutated isogenic CRC cell lines to Cetuximab was investigated. The inventors focused on isogenic cell lines where the p.G12V and p.G13D alleles have been introduced in the genome of human colorectal SW48 cells by targeted homologous recombination (20). The three cell lines were stimulated with EGF to activate the EGFR pathway. The two cell lines carrying the KRAS mutations showed a diminished response to EGF, perhaps because the EGFR pathway is constitutively active. Then, to block the activation of the pathway, cells were treated with Cetuximab, resulting in a decrease of cell proliferation. Although the KRAS-G12V cells were less sensitive to the drug than KRAS-G13D cell, they were still responsive; and since CRC patients carrying the KRAS-G12V mutation do not respond to Cetuximab treatment in the clinic, the results suggested that standard 2D cell culture conditions do not fully model well the correlation between the mutant KRAS and the resistance to the drug *in vitro* (12, 22).

It was concluded that 2D-cell culture does not adequately model the sensitivity and resistance to Cetuximab treatment observed *in vivo,* and in CRC patients.

### Growing spheroids of SW48 cells in ultra-low attachment cell culture flasks in colonosphere medium and studying of their secretomes in both the 2D and 3D formats

The lack of correlation between the resistance to Cetuximab and KRAS mutations in the model system made the inventors explore other experimental setups. It has been recognized that *in vitro* sensitivity to cancer drugs might differ when cell lines are growing in 2D or 3D (23-25).

Thus, the inventors decided to test this possibility by growing spheroids of SW48 cells in ultra-low attachment cell culture flasks in colonosphere medium and study their secretomes (Figure S1). In both the 2D and 3D formats the cells were equally viable, with live cells exceeding 90%, and a low signal for cleaved PARP (apoptotic marker). Once the protocol to generate 3D secretomes was established, the secretomes obtained from the parental CRC cells growing either in 2D or in 3D were compared. The two secretomes were analyzed in triplicate by running 4h chromatographic gradients on LTQ-Orbitrap Velos. An average of 22,826 peptide counts for each of the different technical replicates of the two biological conditions (protein false discovery rate (FDR)under 0.5%) were obtained and a non-redundant list of 1564 proteins was generated for the dataset.

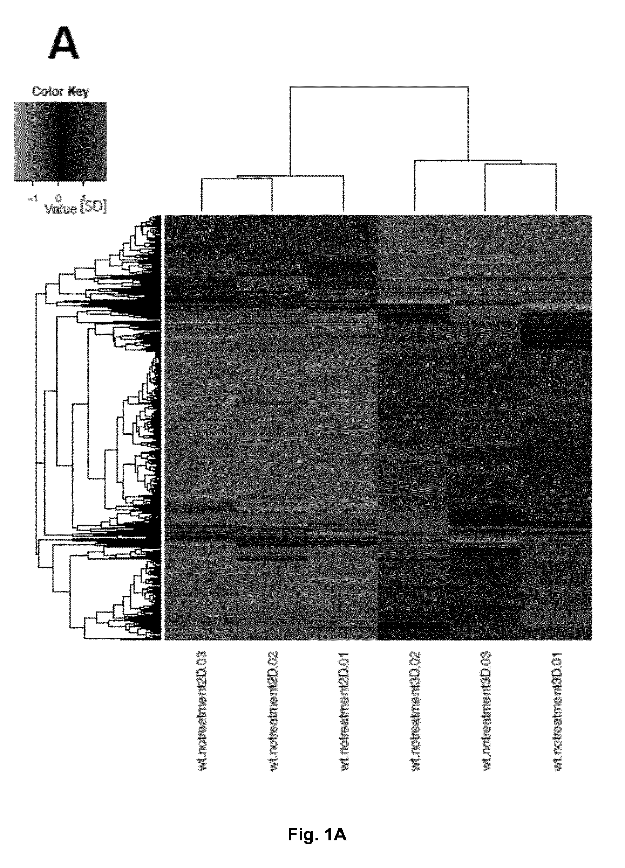

Figure 1A shows the unsupervised hierarchical clustering analysis of the datasets related to the 2D or 3D culture format of SW48 parental cells (first biological replicate). Unsupervised average-linkage hierarchical clustering was done with the spectral count data, following their export from Scaffold software into R. Data were normalized on the basis of secreted protein per cell. The entire protein list was used. Columns represent samples; rows are proteins. The data rows are centered and scaled to 1 sd prior to produce the heat map. This analysis by hierarchical clustering after normalization showed clear-cut differences between the secretomes from the two cell culture formats (Figures 1A).

The inferential analysis of the dataset, using Quasilikelihood (QLLL) GLM and filters (signal _ 4 spectral counts, fold change _1.8 and adjusted p-values _ 0.05), resulted in 849 differentially secreted proteins, when both biological replicates are considered. It can be observed that EGFR is found among these proteins with the following characteristics.

| **Protein Name** | **Gene Name** | **3D Cell Culture Format** | **2D Cell Culture Format** | **LogFC** | **Adjusted p-value** |
|---|---|---|---|---|---|
| EGFR_HUMAN | EGFR | 0 | 7 | -32,54 | 1,07E-08 |

Proteins identified (protein FDR under 0.5%) of the SW48 parental secretome dataset

in the 2D and 3D cell culture formats. EGFR is one of the differentially secreted proteins passing the post-filtering criteria.

All the proteins meeting the highly strict filters were considered, regardless of their theoretical compartment localization. There is a large body of literature supporting nonclassical protein secretion. In this regard the inventors have recently proved that a substantial fraction of the proteins secreted by tumor cells are secreted through alternative pathways to the classical ER-Golgi secretion (16). These proteins find their way out of the cell using different routes, such as protease assisted-shedding or through exosomes and microvesicle exocytosis.

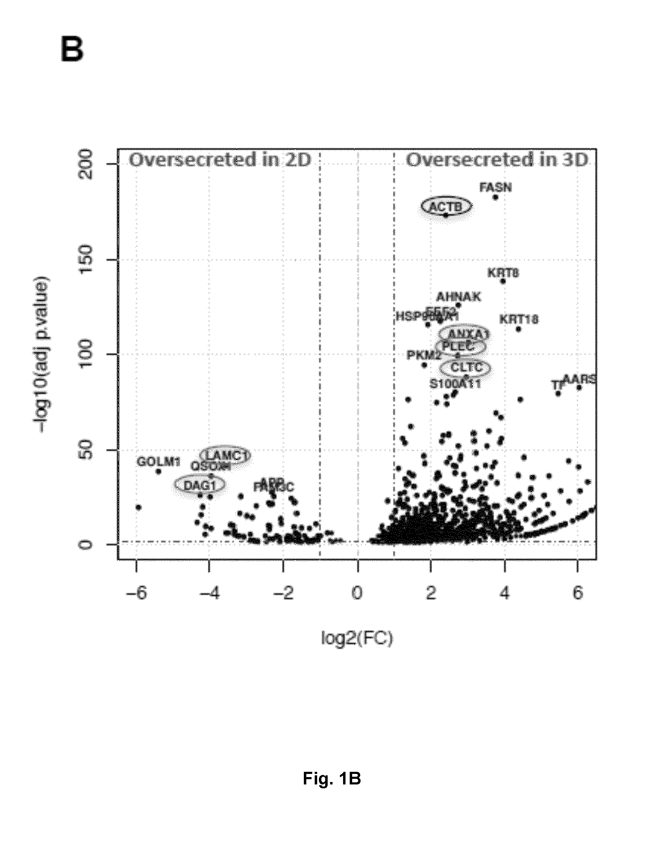

The volcano plot in Figure 1B shows a large proportion of proteins oversecreted in 3D-spheroids as compared to the 2D monolayer (two biological replicates, three technical replicates). The logarithmic ratio of fold change is plotted against the negative logarithmic adjusted p-value. On the right side of the plot are proteins that are significantly over-secreted in 3D. Proteins that are significantly over-secreted in 2D are shown on the left. Circled in red are proteins whose secretions are highly regulated depending on the cell culture format, and are related to cell-to-cell communication, cytoskeleton and vesicle trafficking. CTB (circled in black) was not found highly regulated, when the second biological replicate was considered.

Secretomes of SW48 parental cells in 2D and 3D are significantly different in proteins that reflect CRC biology.

### In vitro investigation of the sensitivity to Cetuximab in the two cell culture formats

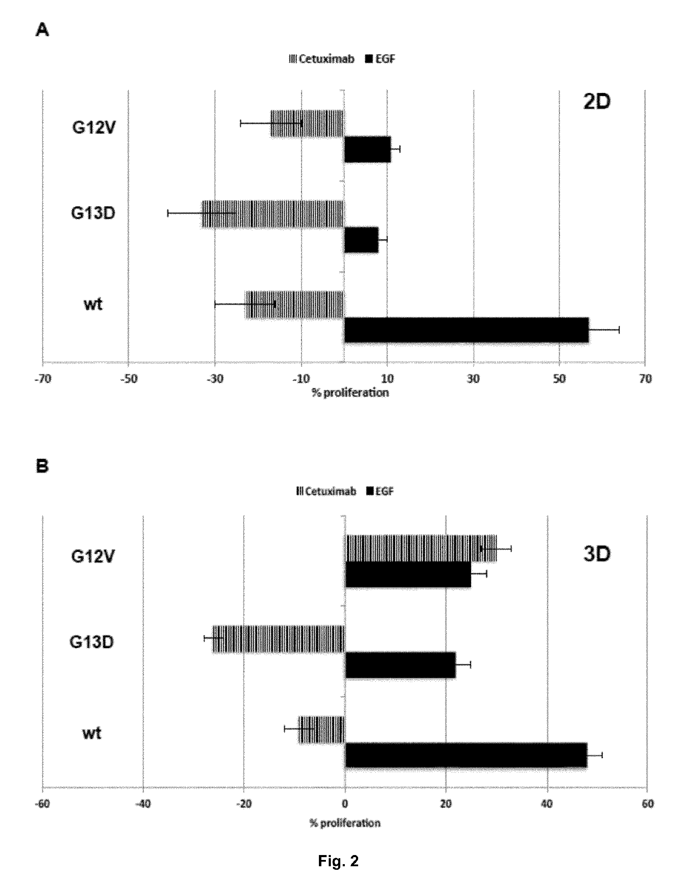

Based on the significant differences obtained between the secretomes of the parental SW48 cells in the 2D versus 3D formats, the *in vitro* sensitivity to Cetuximab in the two cell culture formats was investigated. Hence, the treatment regimens outlined in Figure 2 to all three isogenic cell lines, growing either in 2D or in 3D were applied. Thus, Figure 2 shows the Cetuximab effect on cellular proliferation of SW48 KRAS-wild type and KRAS-mutant isogenic cell lines in 2D and 3D cell culture. The treatment schemes exhibit some modifications when the 3D setting is considered, namely (i) spheroids are grown for five days before secretomes are employed and (ii) the colonosphere medium differs from the DMEM-F12 used in standard 2D culture conditions (Figure S1). Both the EGF and Cetuximab concentrations were optimized for the working conditions based on recently published studies (1,12,22); within a range of tested concentrations, the lower ones that were still able to stimulate (EGF) and block (Cetuximab) the proliferation of SW48 cells were selected. To measure the stimulating and blocking effects on the EGFR pathway on SW48 cells, the percentage of cell proliferation in the EGF and the EGF/Cetuximab conditions was calculated, normalized by the non-treated cells. In 2D, the proliferation of the three cell lines is blocked by Cetuximab, and the KRAS wild-type cells are much more sensitive to the EGF stimulation than the KRAS mutant cells (Figure 2A). Figure 2A shows how Cetuximab inhibits the proliferation of both KRAS-wild type and KRAS-mutant cell lines in 2D. KRAS-wild type, KRAS-G12V and KRAS-G13D cells were serum starved for 18h and treated with 2 ng/mL of EGF or 2 ng/mL of EGF (10 min) and 0.5 mg/mL of Cetuximab for 24h. Non-treated cells served as negative controls for each cell line. Cell viability and % proliferation were determined using Countess (Invitrogen, Carlsbad, CA). Results were normalized to growth of non-treated cells. Experiments were performed, at least, in triplicate. Results are shown as mean ± std.

In 3D, EGF similarly stimulates the proliferation of the three cell lines. However, only the proliferation of the KRAS-wild type and KRAS-G13D cells is blocked by Cetuximab. The proliferation of KRAS-G12V cells is not affected in the presence of Cetuximab (Figure 2B). As described for the 2D setting, KRAS-wild type, KRAS-G12V and KRAS-G13D cells were treated with EGF or EGF/Cetuximab, following serum starvation. Non-treated cells also served as negative controls for each cell line. Cell viability and % proliferation were determined and results were normalized to growth of nontreated cells. Experiments were performed, at least, in triplicate. Results are shown as mean ± std. EGF = epidermal growth factor; wt = KRAS-wild type cells; G12V = KRAS-G12V cells; G13D = KRAS-G13D cells.

The results obtained for the spheroids are in line with those obtained when the same cell lines were grown as xenografts in immunocompromised mice, and more importantly with the results obtained with CRC patients (4, 12, 13).

### Profiling of the secretomes of SW48 KRAS-wild type, KRAS-G13D and KRAS-G12V cells in response to EGF and EGF/Cetuximab (in both 2D and 3D)

Following the 3D model validation, showing drug response profiles in 3D that better recapitulate patient data and differential secretome patterns compared to 2D-conditions, the inventors next determined how drug treatment translates to a secreted molecular response. The secretomes of SW48 KRAS-wild type, KRAS-G13D and KRAS-G12V cells in response to EGF and EGF/Cetuximab (in both 2D and 3D) were profiled. Viability assays and the apoptotic analysis of cells show that SW48 cells maintain high cell viability and low apoptotic signal for 24h in serum-free media, which is not different than that obtained on the same cells growing with 10% serum.

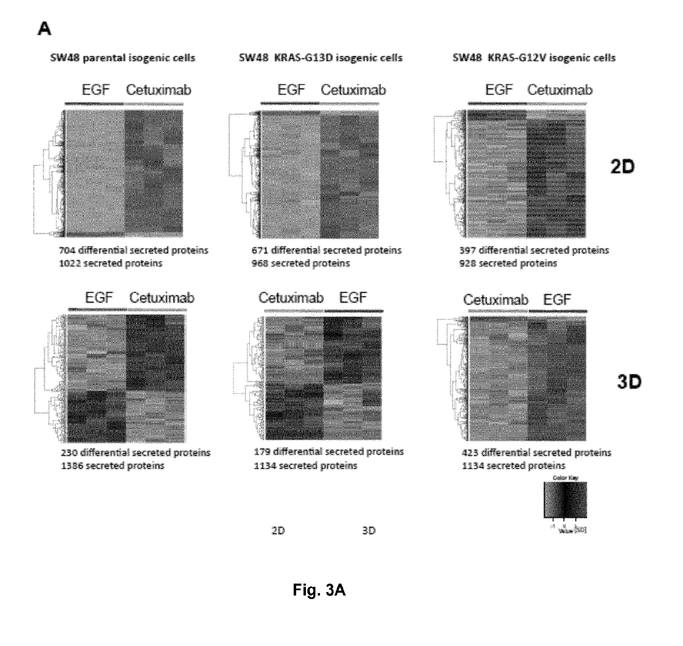

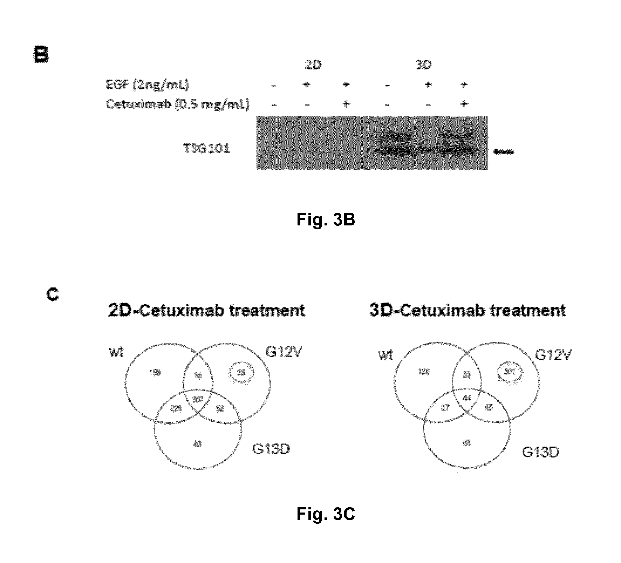

Figure 3 shows the Secretome Proteomics of Sensitivity to Cetuximab in 2D and 3D.

in both biological replicates in all three cell lines (Figures 3A). The secretomes upon treatment in 2D are more dissimilar than in 3D, being in 2D the number of secreted proteins for the treatments with EGFR and Cetuximab very large, whereas in 3D the number of secreted proteins for the treatments with EGFR and Cetuximab is considerably smaller

The 3D secretomes seem to be much more complex than those in 2D. In all three cell lines, the number of proteins identified in the 3D secretomes is consistently greater than in the corresponding 2D secretomes, for the same amount of secretome analyzed (Figure 3A). Part of this additional complexity might come for an observed increase in exosomes present in the 3D secretome of the parental cell line, when compared to 2D (Figure 3B). In Figure 3A heat maps are shown representing the proteins that were significantly over- and/or down secreted, when SW48 parental, KRAS-G13D and KRAS-G12V cells were grown in the 2D or 3D format and treated with EGF or EGF/Cetuximab (first biological replicate). Data analysis was based on spectral count data after exporting it from Scaffold software into R. The GLM model based on the Poisson distribution was used to test significance. The Benjamini & Hochberg multitest correction was employed to adjust the p-values with control on the FDR. The data were normalized on the basis of secreted protein per cell, taking also into account an adjusted significance value of 0.05 and the post-test filters (SpC of 4, LogFC of 0.8), as described in the Experimental procedures section. A high abundance filter of 11 was used on the entire protein list exported from Scaffold software. Columns represent samples; rows are proteins. Red represents proteins that were over-secreted and green represents proteins that were down-secreted in the presence of EGF or EGF/Cetuximab. The data rows are centered and scaled to 1 sd prior to produce the heatmap. Figure 3B shows how equal concentrations of secretomes (15 µg) were resolved by SDS-PAGE and western blotted against TSG101, a stablished exosome marker. TSG101 levels are obtained in the 3D format, but not in 2D, showing differences among treatments and when compared to the non-treated cells.

Despite the complexity of the secretomes being higher in 3D-spheroids, the number of significant differences arising from comparing the treatment with Cetuximab in the three cell lines is much smaller than that in 2D, as can be seen in Figure 3C. Figure 3C shows Venn diagrams illustrating the significant proteins that differ between the two cell culture formats (2D and 3D) upon Cetuximab treatment, when comparing the dataset of KRAS-wild type status to the dataset of KRASG13D and that of KRAS-G12V status. The GLM Poisson test was used for the generation of the Venn diagrams.

When one looks at the unique and shared regulated proteins secreted in the three cell lines upon treatment, the isogenic cell line harboring the KRAS-G12V mutation has a large number of unique secreted proteins regulated in 3D-spheroids as compared to the 2D-cell culture.

Since, the KRAS-G12V mutation confers resistance to Cetuximab in CRC patients and a large decrease in sensitivity to Cetuximab for this cell line growing in 3D was observed, the large number of unique significant proteins secreted in the G12V mutant could be related to the change in drug sensitivity.

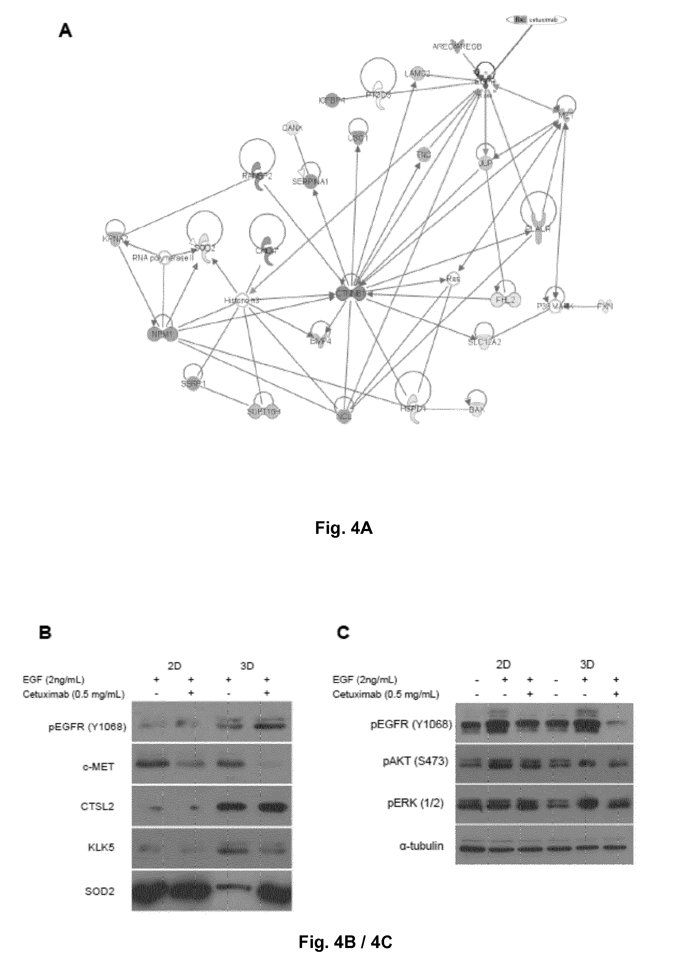

Figure 4 shows how Cetuximab action in SW48 cells differentially regulates the EGFR pathway linked secreted response in 3D-spheroids as compared to the 2D-cell culture.

Since the sensitivity to Cetuximab changes when the 2D setting is compared to the 3D-spheroids, the inventors reasoned that EGFR pathway-related proteins could be differentially regulated in the secretomes of SW48 cells growing in the two culture formats. The analysis of the dataset related to the SW48 KRAS-wild type cells upon treatment in 3D-spheroids resulted in a list of 344 differentially secreted proteins, when both biological replicates are considered. Among said proteins EGFR was found:

| Protein Name | Gene Name | EGF/Cetuximab | EGF | LogFC | Adjusted p-value |
|---|---|---|---|---|---|
| EGFR_HUMAN | EGFR | 5 | 0 | 31,86 | 1,16E-05 |

The complete list was uploaded into the IPA software.

Figure 4A shows the Ingenuity Pathway Analysis of the proteins that are differentially secreted upon EGF and EGF/ Cetuximab treatment of SW48 parental cells in 3D (two biological replicates, three technical replicates). The analysis showed that the top network generated contained two major hubs related to the EGFR pathway: EGFR and beta-catenin. The network contains secretome proteins (in red and green) and gene objects (in white) connected to them by IPA. Green represents secretome proteins whose levels increase upon EGF treatment, whereas red corresponds to secretome proteins whose levels increase upon Cetuximab treatment in 3D.

Since EGFR is the target of Cetuximab and beta-catenin is the main oncogenic driver in CRC initiation, the analysis shows the relevance of the secretome quantitative analysis towards the identification of response biomarkers to anti-EGFR therapy. To compare the molecular secreted response to Cetuximab in 2D versus 3D, the inventors checked whether the proteins regulated upon the action of Cetuximab in spheroids were also regulated in 2D. This check consisted of performing statistics to find the differential secreted proteins by the action of Cetuximab in both 2D and 3D using the data shown in Figure 3A. The methodology is disclosed in (16) and (21).Several proteins related to the EGFR pathway were only regulated in 3D-spheroids or the regulation went to the opposite direction in 2D. The secretion of hundreds of proteins, among them growth factors (JAG1, HDGF, MIF), proteases (procathepsin H) and extracellular matrix proteins (laminins A5, B2, B3 and C2) was regulated, when the KRAS-wild type cells were treated with Cetuximab in 2D, but no clear canonical pathways were enriched, and no direct relationship to the EGFR pathways was established. Selected proteomic data were validated by Western Blot (Figure 4B). Equal concentrations (15 µg) of secretomes were resolved by SDS-PAGE and western blotted against proteins that are differentially secreted upon treatment in 3D, such as pEGFR, c-Met, CTSL2, KLK5 and SOD2.

One of the most striking results is that the Tyr-phosphorylated form of EGFR (pEGFR) is over-secreted upon Cetuximab treatment (figure 4B). Since the inventors are able to detect by WB a molecule corresponding to the entire activated receptor, it is suggested that the receptor is secreted through exosomes or microvesicles (figure 4B). This is in agreement with the fact that a stronger signal for exosomes in the 3D-spheroids than in the 2D-cell culture conditions was detected. To evaluate whether the EGFR-related secretome correlates with the intracellular activation status, the activated form of three major nodes in the EGFR pathway (pEGFR, pERK, pAKT) was profiled. In 3D, the modulation of the pathway when it is stimulated with EGF and blocked by Cetuximab is more finely regulated than in 2D, where pERK (the major downstream signaling effector) is almost constitutively active regardless of extracellular stimulus (EGF or Cetuximab) (Figure 4C). Figure 4C shows a Western-blot analysis of cell lysates showing the activation of the EGFR pathway. Equal concentrations (30 µg) of cell lysates were resolved by SDS-PAGE and western blotted against pEGFR, pAkt and pErk (1/2).

The secretome and pathway activation molecular findings confirm that there is an EGFR-centric differential secretion in line with sensitivity to Cetuximab, depending on the cell culture format.

### Validation of the results by ELISA

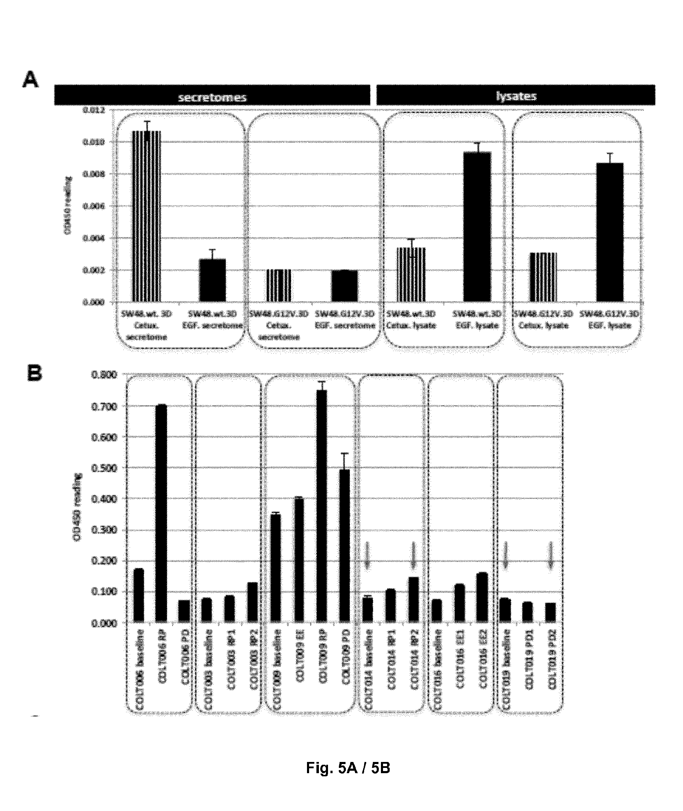

Given the observation that pEGFR is over-secreted upon treatment of KRAS-wt CRC cells with Cetuximab, the results were validated by ELISA. First, pEGFR levels were detected by ELISA in the secretomes of SW48 parental and KRASG12V cells grown in 3D and compared to their counterparts in cell lysates (Figure 5A). Equal concentrations (15 µg) of secretomes and cell lysates were loaded. Experiments were performed, at least, in triplicate. Results are shown as mean ± std. EGF = epidermal growth factor; wt = KRAS-wild type cells; G12V = KRAS-G12V cells. Data agreed with those of proteomics and western blotting, showing an increased pEGFR secretion upon Cetuximab treatment, only in KRAS wild-type cells. Data in lysates confirm the activation/ inhibition of the EGFR pathway. Next, a clinical validation measuring pEGFR in plasma of CRC patients undergoing treatment with Cetuximab was performed. The results suggest that pEGFR is a candidate biomarker of drug response (Figure 5B). Figure 5B shows pEGFR levels detected by ELISA in the plasma of patients undergoing Cetuximab treatment. Results are shown as mean ± std. Experiments were performed, at least, in triplicate. RP = response to Cetuximab (RP1 and RP2 correspond to different dates/ plasma samples; SD = stable disease; PD = progressive disease. The labelled-disease status during treatment (RP, SD, PD) was assessed by standard CT scan-based imaging. Red arrows mark the pEGFR plasma levels corresponding to the CT scan images shown in panel C. Responders or patients with a stable disease show increasing pEGFR levels, whereas disease progression correlates with decreasing pEGFR levels. Noteworthy, patient COLT019 (patient with BRAF mutation) does not show any change in the plasma levels of pEGFR, confirming that the hyperactivation of the EGFR pathway downstream of the receptor-either by KRAS or BRAF mutation- does not increase the secretion of pEGFR.

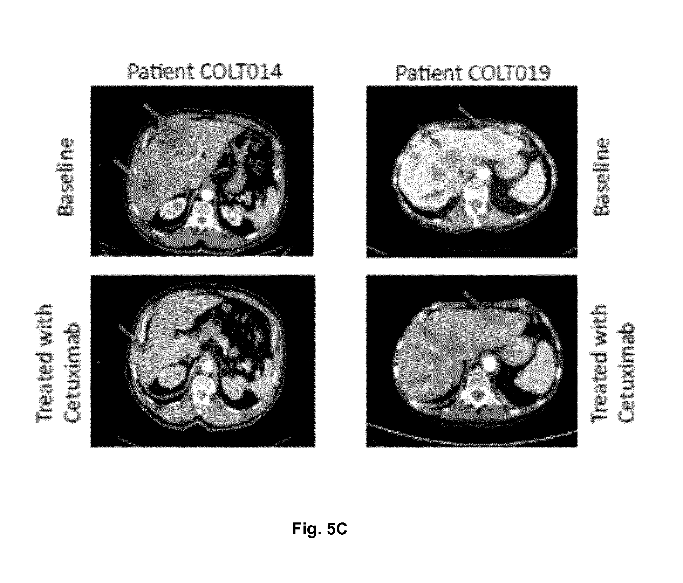

Finally, the CT scan images performed on two of patients, COLT014 and COLT019, is shown in figure 5B (figure 5C) are shown. The imaging of the two patients is shown before (top panels) and after (bottom panels) treatment with Cetuximab. The tumor lesions are marked with red arrows. The CT scans shown correspond to the pEGFR plasma levels marked in panel B with red arrows.

The patient COLT014, who responds to the treatment, shows a clear reduction of the neoplastic lesions, while in the patient with a BRAF mutation (COLT019) Cetuximab treatment has no effect on the tumor lesions. The clinical response to Cetuximab assessed by CT scans is mirrored by the trend in the plasma levels of pEGFR.

### REFERENCES

**1.** Misale S, Yaeger R, Hobor S, Scala E, Janakiraman M, Liska D, et al. Emergence of KRAS mutations and acquired resistance to anti-EGFR therapy in colorectal cancer. Nature 2012; 486: 532-36.
**2.** Diaz L.A. Jr, Williams RT, Wu J, Kinde I, Hecht JR, Berlin J, et al. The molecular evolution of acquired resistance to targeted EGFR blockade in colorectal cancers. Nature 2012; 486: 537-40.
**3.** Villarreal L, Mendez O, Salvans C, Gregori J, Baselga J, Villanueva J. Unconventional Secretion Is a Major Contributor of Cancer Cell Line Secretomes. Mol Cell Proteomics 2013; 12:1046-60.
**4.** Bokemeyer C, Bondarenko I, Hartmann JT, De Braud FG, Volovat C, et al. KRAS status and efficacy of first-line treatment of patients with metastatic colorectal cancer (mCRC) with FOLFOX with or without Cetuximab: The OPUS experience. J Clin Oncol (meeting abstracts) 2008; 26: 4000.
**5.** De Roock W, Piessevaux H, De Schutter J, Janssens M, De Hertogh G, Personeni N, et al. KRAS wild-type state predicts survival and is associated to early radiological response in metastatic colorectal cancer treated with Cetuximab. Ann Oncol 19, 2008, 508-15.
**6.** Di Fiore F, Charbonnier, F., Lefebure, B., Laurent, M., Le Pessot, F., Michel, P., and Frebourg, T. Clinical interest of KRAS mutation detection in blood for anti-EGFR therapies in metastatic colorectal cancer. Br J Cancer 2008; 99: 551-52.
**7.** Karapetis CS., Khambata-Ford S, Jonker DJ, O'Callaghan CJ, Tu D, et al. K-ras mutations and benefit from Cetuximab in advanced colorectal cancer. N Engl J Med 2008; 359: 1757-65.
**8.** Keller A, Nesvizhskii Al, Kolker E, Aebersold R. Empirical statistical model to estimate the accuracy of peptide identifications made by MS/MS and database search. Anal. Chem. 2002; 74: 5383-92.
**9.** Lièvre A, Bachet JB, Boige V, Cayre A, Le Corre D, Buc, E, et al. KRAS mutations as an independent prognostic factor in patients with advanced colorectal cancer treated with Cetuximab. J Clin Oncol 2008; 26: 374-79.
**10.** Nesvizhskii Al, Keller A, Kolker E, Aebersold R. A statistical model for identifying proteins by tandem mass spectrometry. Anal. Chem. 2003; 75: 46-58.
**11.** Allegra CJ, Jessup JM, Somerfield MR, Hamilton SR, Hammond EH, Hayes DF, et al. American Society of Clinical Oncology provisional clinical opinion: testing for KRAS gene mutations in patients with metastatic colorectal carcinoma to predict response to anti-epidermal growth factor receptor monoclonal antibody therapy. J Clin Oncol 2009; 27: 2091-96.
**12.** De Roock W, Jonker DJ, Di Nicolantonio F, Sartore-Bianchi A, Tu D, Siena S, et al. Association of KRAS p.G13D mutation with outcome in patients with chemotherapy-refractory metastatic colorectal cancer treated with Cetuximab. JAMA 2010; 304:1812-20.
**13.** Tejpar S, Celik I, Schlichting M, Sartorius U, Bokemeyer C, Van Cutsem E. Association of KRAS G13D tumor mutations with outcome in patients with metastatic colorectal cancer treated with first-line chemotherapy with or without Cetuximab. J Clin Oncol 2012; 30: 3570-77.
**14.** Rifai N, Gillette MA, Carr S. Protein biomarker discovery and validation: the long and uncertain path to clinical utility. Nat Biotechnol 2006; 24: 971-83.
**15.** Chambers JM. Software for data analysis: Programming with R. New York: Springer-Verlag; 2008.
**16.** Villarreal L, Méndez O, Salvans C, Gregori J, Baselga J, Villanueva J. Unconventional Secretion is a Major Contributor of Cancer Cell Line Secretomes. Mol Cell Proteomics 2013; 12:1046-60.
**17.** Mathias RA, Wang B, Ji H, Kapp EA, Moritz RL, Zhu HJ, Simpson RJ. Secretome-based proteomic profiling of Ras-transformed MDCK cells reveals extracellular modulators of epithelial-mesenchymal transition. J Proteome Res 2009; 8: 2827-37.
**18.** Fijneman RJ, de Wit M, Pourghiasian M, Piersma SR, Pham TV, Warmoes MO, et al. Proximal fluid proteome profiling of mouse colon tumors reveals biomarkers for early diagnosis of human colorectal cancer. Clin Cancer Res. 2012; 18: 2613-24.
**19.** Agresti A. Analysis of Ordinal Categorical Data. New Jersey: John Wiley & Sons Inc; 2010.
**20.** Di Nicolantonio F, Arena S, Gallicchio M, Zecchin D, Martini M, Flonta SE, et al. Replacement of normal with mutant alleles in the genome of normal human cells unveils mutation-specific drug responses. Proc Natl Acad Sci USA 2008; 105: 20864-69.
**21.** Gregori J, Villarreal L, Méndez O, Sánchez A, Baselga J, Villanueva J. Batch effects correction improves the sensitivity of significance tests in spectral counting-based comparative discovery proteomics. J Proteomics 2012; 75: 3938-51.
**22.** Derer S, Berger S, Schlaeth M, Schneider-Merck T, Klausz K, Lohse S, et al. Oncogenic KRAS impairs EGFR antibodies' efficiency by C/EBP_- dependent suppression of EGFR expression. Neoplasia 2012; 14:190-205.
**23.** Luca AC, Mersch S, Deenen R, Schmidt S, Messner I, Schäfer KL, et al. Impact of the 3D Microenvironment on Phenotype, Gene Expression, and EGFR Inhibition of Colorectal Cancer Cell Lines. PLoS One 2013; 8: e59689
**24.** Fang DD, Kim YJ, Lee CN, Aggarwal S, McKinnon K, Mesmer D, et al. Expansion of CD133(+) colon cancer cultures retaining stem cell properties to enable cancer stem cell target discovery. Br J Cancer 2010; 102:1265-75.
**25.** Zou J, Yu XF, Bao ZJ, Dong J. Proteome of human colon cancer stem cells: a comparative analysis. World J Gastroenterol 2011; 17: 1276-85.
**27.** Tabernero J, Cervantes A, Rivera F, Martinelli E, Rojo F, von Heydebreck A, et al. Pharmacogenomic and Pharmacoproteomic Studies of Cetuximab in Metastatic Colorectal Cancer: Biomarker Analysis of a Phase I Dose-Escalation Study. J Clin Oncol 2010; 28: 1181-89.
**28.** Barderas R, Mendes M, Torres S, Bartolomé RA, López-Lucendo M, Villar-Vázquez R, et al. In-depth characterization of the secretome of colorectal cancer metastatic cells identifies key proteins in cell adhesion, migration, and invasion. Mol Cell Proteomics 2013; 12: 1602-20.
**29.** Benjamini Y, Hochberg Y. Controlling the False Discovery Rate: a Practical and Powerful Approach to Multiple Testing. J. Roy. Stat. Soc B Met. 1995; 57:289-300.

## Claims

1. An *in vitro* method for monitoring the response and resistance to a treatment of a patient suffering from an EGFR expressing tumor, with an anti-cancer agent against an anti-EGFR expressing tumor, the method comprising:
- determining the level of pEGFR in a body fluid sample taken from a patient suffering from an EGFR expressing tumor, and
- comparing said level with a reference value, the reference value being the level of pEGFR in a sample taken from the same patient before starting said treatment, and wherein an increase in said level is indicative of response to the treatment, and wherein a decrease in said level is indicative of the appearance of resistance to the treatment.

2. An in vitro method according to claim 1, wherein the anti-cancer agent is an agent with the capacity of inhibiting the tyrosine kinase activity of a protein receptor.

3. An in vitro method according to claim 2, wherein the anti-cancer agent is a monoclonal antibody or a fragment of said monoclonal antibody.

4. An in vitro method according to claim 3, wherein the monoclonal antibody is Cetuximab.

5. An *in vitro* method according to any of claims 1 to 4, wherein the EFGR expressing tumor is selected from the group comprising head and neck, ovarian, cervical, bladder, oesophageal, gastric, breast, endometrial, non-small cell lung cancer and colorectal cancer.

6. An *in vitro* method according to claim 5, wherein the EFGR expressing tumor is colorectal cancer.

7. An *in vitro* method according to any of claims 1 to 6, wherein the sample is selected from the group comprising saliva, urine, interstitial tumor fluid, cerebrospinal fluid, blood, serum, plasma, or derivatives of any of them.

8. An *in vitro* method according to claim 7, wherein the sample is a blood derived sample, preferably a plasma sample.

9. An *in vitro* method according to any of claims 1 to 8, further comprising taking a decision on the treatment with the anti-cancer agent, wherein the treatment is continued if the level of pEGFR is at least as high as the reference value, or the treatment is discontinued if the level of pEGFR is lower than the reference value.

10. An *in vitro* method according to any of claims 1 to 9, wherein pEGFR is EGFR phosphorylated in, at least, position Tyr 1068.

11. A kit for *in vitro* monitoring the response or resistance to a treatment of a patient suffering from an EGFR expressing tumor, with an anti-cancer agent, said kit comprising reagents suitable for determining the level of pEGFR in a body fluid sample taken from said patient.

12. A kit according to claim 11, wherein the sample is a blood derived sample, preferably a plasma sample, the anti-cancer agent is Cetuximab and the cancer is CRC.

13. Use of a kit as defined in claim 11 or 12 for *in vitro* monitoring the response or resistance to a treatment of a patient suffering from an EGFR expressing tumor, with an anti-cancer agent according to the method defined in any of claims 1 to 10.

14. Use of a kit according to claim 13, for *in vitro* monitoring the response or resistance to a treatment of a patient suffering from CRC with Cetuximab by determining the level of pEGFR in a plasma sample taken from said patient and comparing said level with a reference value, the reference value being the level of pEGFR in a sample taken from the same patient before starting said treatment, and wherein the decrease in said level is indicative of the appearance of resistance to the treatment.

15. A reagent for its use in detecting pEGFR in a plasma sample with the kit defined in any of claims 11 or 12 through the *in vitro* method of the invention, as defined in any of claims 1 to 10.
